# EUROPEAN PATENT APPLICATION

(11) **EP 3 141 241 A1**
(43) Date of publication of application: **15.03.2017**
(21) Application number: 16187717.0
(22) Date of filing: 07.09.2016
(51) Int. Cl.: A61K 9/00, A61K 9/16, A61K 31/00

(54) **SUSTAINED RELEASE LOW DOSE FORMULATIONS AND USES THEREOF**

(30) Priority: 08.09.2015 US 201562215248 P
(71) Applicant: Voigt, Andreas, 12621 Berlin (DE); Assogba-zandt, Annette, 10243 Berlin (DE); Reiff, Andreas, San Marino, CA 91108 (US); Hampton, Scott, Tarpon Springs, FL 34689 (US)
(72) Inventor: Voigt, Andreas, 12621 Berlin (DE); Assogba-zandt, Annette, 10243 Berlin (DE); Reiff, Andreas, San Marino, CA 91108 (US); Hampton, Scott, Tarpon Springs, FL 34689 (US)
(74) Representative: Schiweck, Weinzierl & Koch Patentanwälte Partnerschaft mbB

(57) **Abstract**

The present invention belongs to the field of sustained drug release, and provides drug delivery compositions and methods of manufacturing drug delivery compositions.

## Description

### FIELD OF THE INVENTION

The present invention belongs to the field of sustained drug release, and provides drug delivery compositions and methods of manufacturing drug delivery compositions.

### BACKGROUND OF THE INVENTION

Naloxone and naltrexone are opioid receptor antagonists. The need for a long-acting opioid receptor antagonist as a treatment for addiction, by blocking the euphoric effects of illicit opioids for an extended period of time, motivated the development of naltrexone. This antagonist has good oral bioavailability, a long duration of action, and twice the potency of a similar compound called naloxone. Naloxone produces essentially no dysphoria but it has a short duration of action and poor oral bioavailability due to high first-pass hepatic metabolism. The half-life of naltrexone can be up to fourteen (14) hours (oral) and the effect can last for a day while naloxone's half-life is only about 30-81 min. (IV, IM) and only lasts for about 1-4 hours.

The difficult-to-treat, chronic pain disorder, fibromyalgia syndrome (FMS) is characterized by diffuse musculoskeletal pain, fatigue, sleep disturbance, and cognitive impairments. Headaches, stomach problems, and a number of other symptoms are also frequently reported. FMS affects 2-8% of the population and predominately women in the United States. The disorder can be debilitating, as the pain and fatigue prevent the individual from carrying out their normal activities.

FMS is seen as a lifelong disease that rarely resolves completely. The FDA has approved four medications for FMS including Amitriptyline (Elavil®), pregabalin (Lyrica®), milnacipran (Savella®), and duloxetine (Cymbalta®), but not all individuals respond well to those treatments. In fact, it is estimated that only about a third of the patients currently respond to these medications. Due to these shortcomings in safety and tolerability of conventional therapies there remains a large clinical unmet need.

One agent, as a potential alternative to naltrexone, is acamprosate (Campral), which may help alcoholics maintain abstinence by preventing relapse. Acamprosate has in vitro affinity for GABA type A and GABA type B receptors, so it's been assumed that the therapeutic effects of acamprosate are due to actions on GABA receptors. However, acamprosate does not share most of the other effects of GABA receptor modifying drugs, such as antianxiety, hypnotic, or muscle relaxant activity. Acamprosate is structurally related to 1-glutamic acid (1-glutamate), which is an excitatory neurotransmitter. It has not been extensively studied in chronic pain and cannot be used in patients with chronic renal disease.

In view of the above, there is a need for improving drug-delivery methods and compositions.

### SUMMARY OF THE INVENTION

According to one embodiment, the present invention provides a drug delivery composition, comprising a microparticulate formulation, said microparticulate formulation further comprising a matrix, a solvent, and at least one low dose opioid receptor antagonist or a combination thereof, wherein said opioid receptor antagonist is incorporated into said matrix homogenously or substantially homogenously.

According to another embodiment, the matrix controls release of said opioid receptor antagonist for initial release rate, daily release rate, duration, and completeness of release, allows release of exact amount of the opioid receptor antagonist, allows sustained release of the opioid receptor antagonist, or a combination thereof.

According to another embodiment, the matrix comprises a solid hydrophobic component and a liquid hydrophobic component.

According to another embodiment, the solvent is phosphate buffered saline or water.

According to another embodiment, the low dose opioid receptor antagonist is selected from group consisting of naltrexone and naloxone.

According to another embodiment, the microparticulate formulation is in a powder form or an injectable form.

According to another embodiment, the present invention provides a method of manufacturing a drug delivery composition, comprising mixing the solid hydrophobic component and the liquid hydrophobic component of the matrix and the opioid receptor antagonist until a microparticulate formulation in a powder form is formed; adding a small amount of water or phosphate buffered saline; and mixing continuously to incorporate the opioid receptor antagonist homogenously or substantially homogenously into the matrix.

According to another embodiment, a method of manufacturing the drug delivery composition comprises adding the solid hydrophobic component and the liquid hydrophobic component of the matrix; mixing said components by kneading, pressing, folding or combination thereof to obtain a kneadable macroscopic mass; combining the macroscopic mass with the opioid receptor antagonist; mixing the macroscopic mass with the opioid receptor antagonist by kneading, pressing, folding or a combination thereof to incorporate the opioid receptor antagonist homogenously into the matrix, thereby payloading the matrix; micronizing the payloaded matrix by adding magnesium stearate to the payloaded matrix and mixing the payloaded matrix and magnesium stearate by kneading, pressing, folding or a combination thereof until a microparticulate formulation in a powder form is formed.

According to another embodiment, a method of manufacturing the drug delivery composition further comprises fractionating the microparticulate formulation by sieving.

According to another embodiment, a method of manufacturing the drug delivery composition further comprises adding the solid hydrophobic component and liquid hydrophobic component of the matrix and the opioid receptor antagonist in a reaction vessel, wherein the solid hydrophobic component is added in a two-fold amount to the mixture; and mixing the components and the opioid receptor antagonist by kneading, pressing, folding, grinding, milling, or a combination thereof so that the microparticulate formulation has a size and size distribution that allows it to be administered as an injectable.

According to another embodiment, a method of manufacturing the drug delivery composition further comprises fractioning the microparticulate formulation by sieving. According to yet another embodiment, said microparticulate formulation is resuspended in an aqueous or an oily suspension. According to yet another embodiment, the microparticulate formulation is prefilled in a syringe in powder form or dissolved in aqueous solution outside of the syringe and then used to fill the syringe. According to yet another embodiment, the prefilled syringe further comprises at least one of a biocompatible oil, saline, and saline with hyaluronic acid.

According to another embodiment, the present invention provides a method of treating an individual with a chronic pain disorder, comprising administering the drug delivery composition to the individual. According to yet another embodiment, the drug delivery composition is administered intramuscularly, intravenously, intradermally, subcutaneously, or via another route.

According to yet another embodiment, administration of the drug delivery composition allows sustained release of the opioid receptor antagonist in the individual's body.

According to another embodiment, the present invention provides a method of treating an individual suffering from or that has been diagnosed with fibromyalgia, Crohn's disease, multiple sclerosis, and pruritus associated with systemic sclerosis.

### BRIEF DESCRIPTION OF THE DRAWINGS

The accompanying drawings are included to further an understanding of the embodiments that are incorporated in and constitute a part of this specification. The drawings illustrate embodiments and together with the description serve to explain principles of embodiments. Other embodiments and many of the intended advantages of embodiments will be readily appreciated, as they become better understood by reference to the detailed description. The elements of the drawings are not necessarily to scale relative to each other.
**Figure 1** shows a representative high quality calibration curve for naltrexone base.
**Figure 2** shows sample profile characteristics for release of naltrexone base from a matrix formulation according to the present invention.
**Figure 3A** shows data for naltrexone release from macroscopic matrices S-220.2 vs. S-221.2.
**Figure 3B** shows data for naltrexone release from macroscopic matrices S-222.2 vs. S-223.2.
**Figure 4A** shows data for naltrexone release from macroscopic matrices S-224.2, S224.3 and 224.4.
**Figure 4B** shows data for naltrexone release from macroscopic matrices S-225.2, S225.3 and 225.4.
**Figure 5A** shows data for naltrexone release from macroscopic matrices S-226.2, S226.3 and 226.4.
**Figure 5B** shows data for naltrexone release from macroscopic matrix S-228.2.
**Figure 6A** shows data for naltrexone release from macroscopic matrix S-229.2.
**Figure 6B** shows data for naltrexone release from macroscopic matrices S-230.2, S230.3 and 230.4.
**Figure 7A** shows data for naltrexone release from micronized matrices D-130.1.1, D-130.1.2, D-130.1.3, D-130.2.1, D-130.2.2 and D-130.2.3.
**Figure 7B** shows data for naltrexone release from micronized matrices D-131.1, D-131.2, and D-131.3.
**Figure 8** shows data for naltrexone release from micronized matrices S-238.1.1, S-238.1.2, S-238.1.3, S-238.2.1, S-238.2.2 and S-238.2.3.
**Figure 9** shows representative data for naltrexone release from melted stearate matrices A-009.1 and A-009.2
**Figure 10** shows data for naltrexone release from micronized matrices S-238.1 and 238.2 (payload 15% naltrexone, approximately).
**Figure 11** shows release of naltrexone base from a matrix using cottonseed oil vs. water as a solvent to decrease needle gauge.

### DESCRIPTION OF PREFERRED EMBODIMENTS OF THE INVENTION

The following language and descriptions of certain preferred embodiments of the present invention are provided in order to further an understanding of the principles of the present invention. However, it will be understood that no limitations of the present invention are intended, and that further alterations, modifications, and applications of the principles of the present invention are also included.

In general, the present invention is drawn to a novel, drug delivery platform technology that allows the sustained release delivery of active agents or drugs, without any chemical modification of the drug. The present invention provides significant and unexpected advantages given that current commercially available drug delivery systems have significant limitations. In addition, the technology described herein is also tunable, controlling drug release for initial release rate (burst rate), daily release rate (first derivation of release profile), duration and completeness of release.

Further, unlike many other drug delivery systems, the technology described herein can be put into a standard syringe and injected under the skin (subcutaneously) through a 28 G needle or into a muscle (intramuscularly) using a needle of 22 gauge or smaller and with a volume of less than three (3) milliliters. The matrix systems described herein can be fine-tuned to release the exact amount of drug that is needed on a daily basis avoiding the risk of overdosing and other side effects, and with substantially linear kinetics.

As used herein, with regard to the present invention, "low-dose naltrexone" (LDN) describes the "off-label" use of naltrexone at low doses for diseases not related to chemical dependency or intoxication. Naltrexone, given at low dosages (in the range of about 3-5 mg/day), has been demonstrated to reduce symptom severity in a small number of chronic conditions, including fibromyalgia, Crohn's disease, multiple sclerosis, and pruritus associated with systemic sclerosis. As an orally available compound that is structurally similar to naloxone, naltrexone may work to reduce disease severity by enhancing endogenous endorphin function and suppressing centrally acting proinflammatory cytokines, thereby helping to attenuate pain and other symptoms. This antiinflammatory effect is distinct from the better-known effect of naltrexone in the blockade of neuronal opioid receptors and may instead involve the antagonism of immune cell receptors, including microglia in the central nervous system. As used herein, with regard to the present invention, "low-dose naltrexone" (LDN) is further intended to refer to novel and non-obvious "off-label" uses of naltrexone at low doses for diseases not related to chemical dependency or intoxication, where there is a significant unmet need for effective treatments.

For the purpose of this specification, the term "mixing" intends to describe, by way of non-limiting example, a mechanical process or a mechanical treatment of the components. Further, by way of non-limiting example, mixing can be in the sense of carrying out repeated cycles of pressing and folding or comparable processing steps which lead to an intense compression and mixing of the components.

In addition to the uses of the matrix systems as described herein for release of naltrexone, the matrix systems of the present invention can reliably and predictably be used for release of naloxone, and for achieving release of the exact amount of naloxone that is needed or desired.

According to the present invention, because the drugs are incorporated into the matrix homogeneously, or substantially homogeneously, even sudden matrix destruction would have a rather moderate effect on release modification. This technology has broad application for multiple biologic agents, small molecule inhibitors, peptides, and low molecular weight drugs.

The present invention provides novel, non-obvious, surprising and unexpectedly beneficial formulations that reliably provide sustained release of an active pharmaceutical agent, such as naltrexone or naloxone, and representative processes and procedures to prepare and obtain such formulations.

According to one embodiment, the present invention provides a drug delivery composition, comprising a microparticulate formulation, said microparticulate formulation further comprising a matrix, a solvent, and at least one low dose opioid receptor antagonist or a combination thereof, wherein said opioid receptor antagonist is incorporated into said matrix homogenously or substantially homogenously.

According to another embodiment, the matrix controls release of said opioid receptor antagonist for initial release rate, daily release rate, duration, and completeness of release, allows release of exact amount of the opioid receptor antagonist, allows sustained release of the opioid receptor antagonist, or a combination thereof

According to another embodiment, the matrix comprises a solid hydrophobic component and a liquid hydrophobic component.

According to another embodiment, the solvent is phosphate buffered saline or water.

According to another embodiment, the low dose opioid receptor antagonist is selected from group consisting of naltrexone and naloxone.

According to another embodiment, the microparticulate formulation is in a powder form or an injectable form.

According to another embodiment, the present invention provides a method of manufacturing a drug delivery composition, comprising mixing the solid hydrophobic component and the liquid hydrophobic component of the matrix and the opioid receptor antagonist until a microparticulate formulation in a powder form is formed; adding a small amount of water or phosphate buffered saline; and mixing continuously to incorporate the opioid receptor antagonist homogenously or substantially homogenously into the matrix.

According to another embodiment, a method of manufacturing the drug delivery composition comprises adding the solid hydrophobic component and the liquid hydrophobic component of the matrix; mixing said components by kneading, pressing, folding or combination thereof to obtain a kneadable macroscopic mass; combining the macroscopic mass with the opioid receptor antagonist; mixing the macroscopic mass with the opioid receptor antagonist by kneading, pressing, folding or a combination thereof to incorporate the opioid receptor antagonist homogenously into the matrix, thereby payloading the matrix; micronizing the payloaded matrix by adding magnesium stearate to the payloaded matrix and mixing the payloaded matrix and magnesium stearate by kneading, pressing, folding or a combination thereof until a microparticulate formulation in a powder form is formed.

According to another embodiment, a method of manufacturing the drug delivery composition further comprises fractionating the microparticulate formulation by sieving.

According to another embodiment, a method of manufacturing the drug delivery composition further comprises adding the solid hydrophobic component and liquid hydrophobic component of the matrix and the opioid receptor antagonist in a reaction vessel, wherein the solid hydrophobic component is added in a two-fold amount to the mixture; and mixing the components and the opioid receptor antagonist by kneading, pressing, folding, grinding, milling, or a combination thereof so that the microparticulate formulation has a size and size distribution that allows it to be administered as an injectable.

According to another embodiment, a method of manufacturing the drug delivery composition further comprises fractioning the microparticulate formulation by sieving. According to yet another embodiment, said microparticulate formulation is resuspended in an aqueous or an oily suspension. According to yet another embodiment, the microparticulate formulation is prefilled in a syringe in powder form or dissolved in aqueous solution outside of the syringe and then used to fill the syringe. According to yet another embodiment, the prefilled syringe further comprises at least one of a biocompatible oil, saline, and saline with hyaluronic acid.

According to another embodiment, the present invention provides a method of treating an individual with a chronic pain disorder, comprising administering the drug delivery composition to the individual. According to yet another embodiment, the drug delivery composition is administered intramuscularly, intravenously, intradermally, subcutaneously, or via another route.

According to yet another embodiment, administration of the drug delivery composition allows sustained release of the opioid receptor antagonist in the individual's body.

According to another embodiment, the present invention provides a method of treating an individual suffering from or that has been diagnosed with fibromyalgia, Crohn's disease, multiple sclerosis, and pruritus associated with systemic sclerosis.

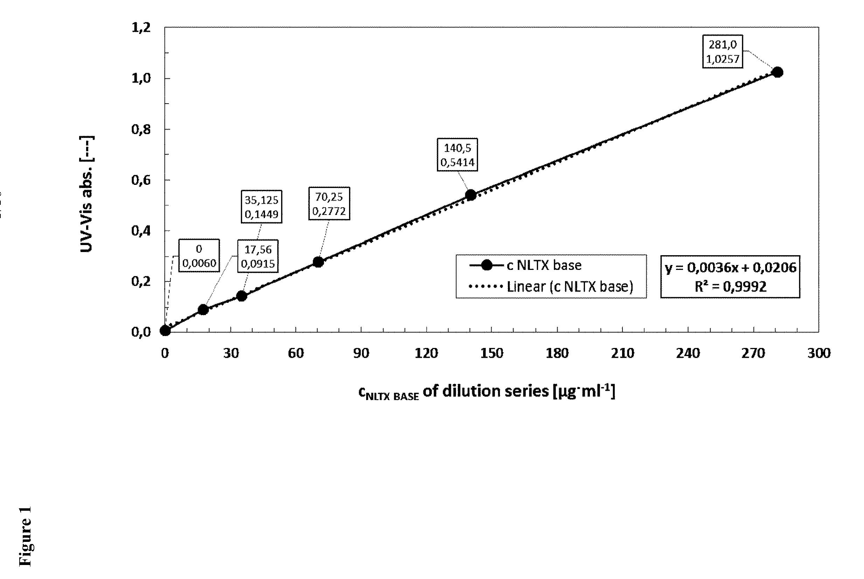

Now referring to the Figures, by way of non-limiting example, **Figure 1** shows a representative high quality calibration curve for the naltrexone base.

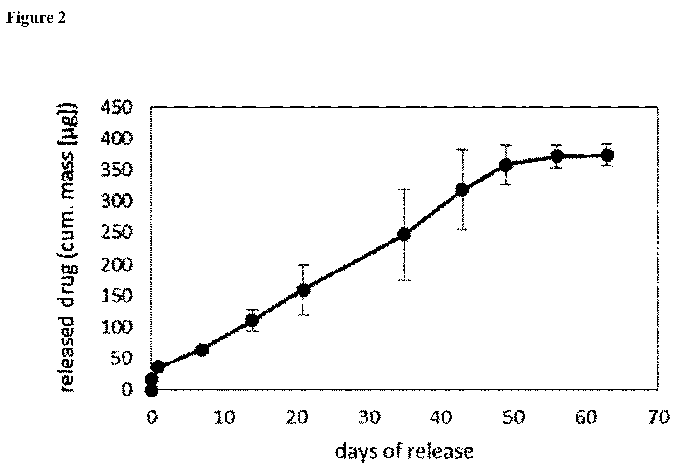

Referring to another non-limiting example, **Figure 2** shows sample profile characteristics for release of naltrexone base from a matrix formulation according to the present invention. In particular, **Figure 2** shows a representative curve for sample release of naltrexone base from a matrix ("A-050 matrix" in this study), in which the matrix comprises magnesium stearate and tocopherol in a ratio of about 87:13, with an approximate 30% naltrexone payload. Before release was measured, steps had been taken to incorporate (payload) naltrexone into the matrix. The number of days of release are shown on the horizontal axis (X axis). The cumulative mass of released naltrexone is shown on the vertical axis (Y axis). The circles along the release curve represent release data from the matrix, measured at different days of release. The matrix provides very reliable sustained release of low-dose naltrexone.

The release profile of any low-dose formulation, in accordance with the present invention, can be controlled by several factors including but not limited to, for example, variations of the payload, size and distribution of the matrix, and selection of the excipients.

To achieve a desired result for administration of a low-dose formulation to an individual, the present invention also contemplates that other parameters may be adjusted as needed or desired, including but not limited to injection volume, injection type, syringe type and size, and needle gauge.

For purposes of illustration of the present invention, sample naltrexone release data from several different representative macroscopic matrices and micronized matrices are shown and described below.

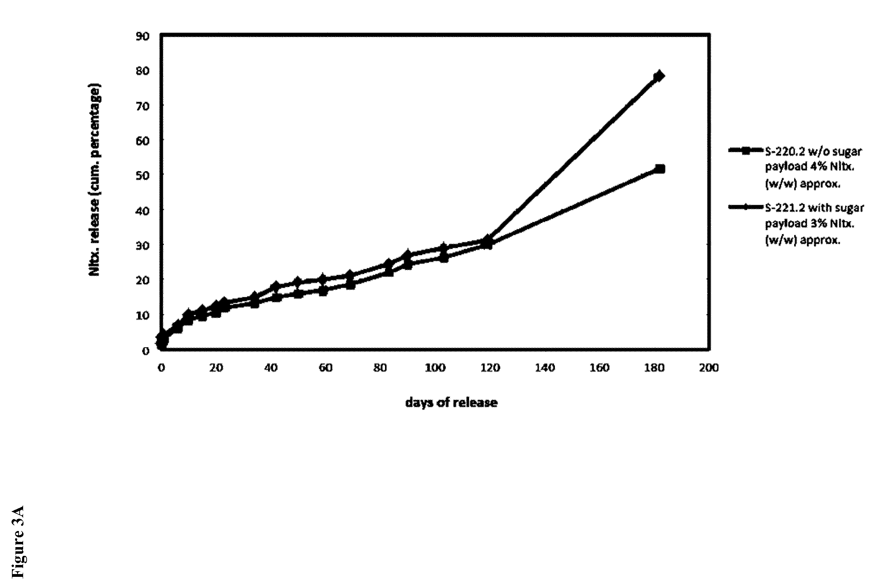

Referring to a non-limiting example, **Table 1** provides a summary of representative macroscopic matrices, wherein each matrix comprises a naltrexone payload. **Table 1** shows specific sample ID numbers for each macroscopic matrix that comprises a naltrexone payload. By way of non-limiting example, **Figures 3A****,** **3B****,** **4A****,** **4B****,** **5A****,** **5B****,** **6A** **and** **6B** show corresponding release data for these sample ID numbers listed in **Table 1.**

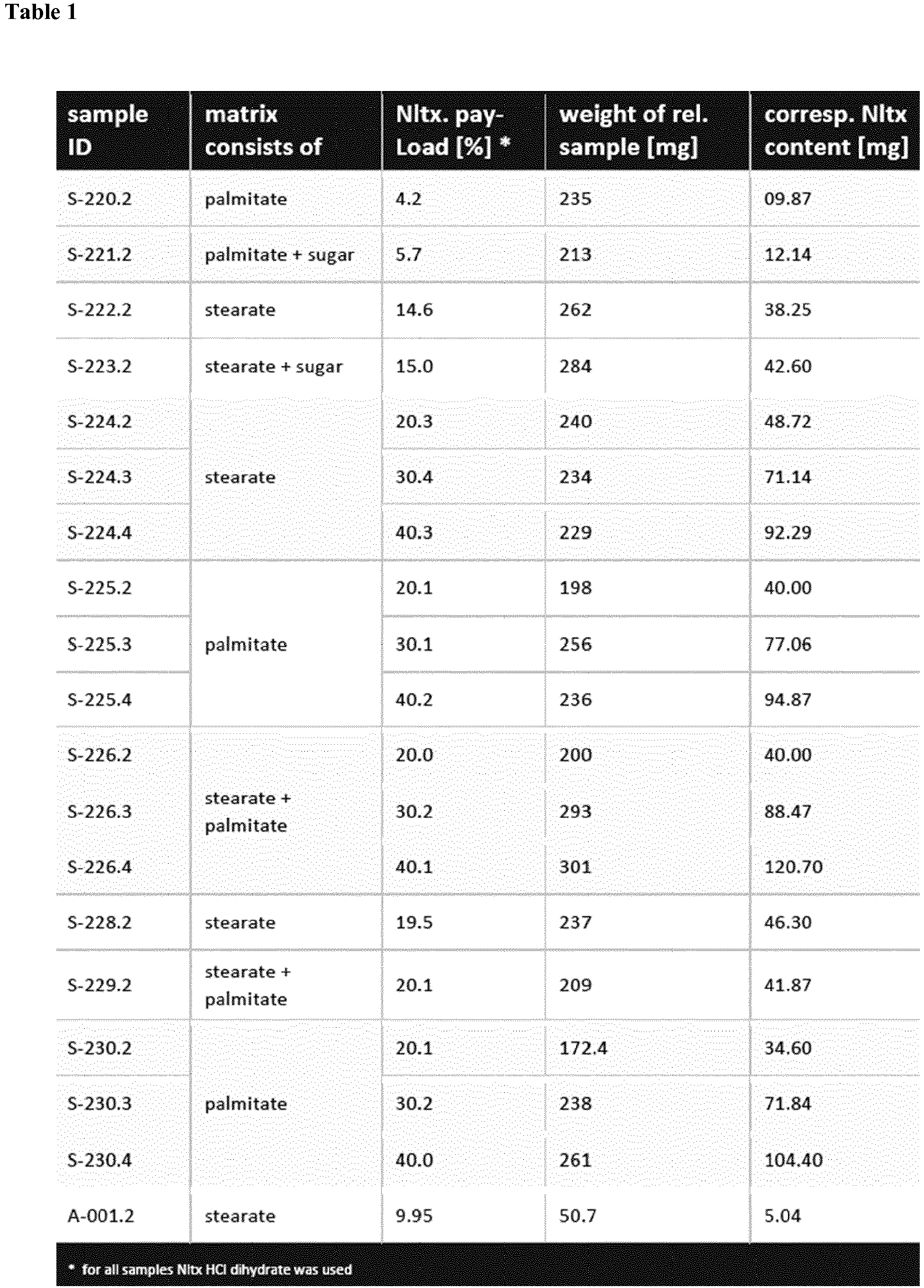

Referring to **Figure 3A****,** this figure shows sample profile characteristics for naltrexone release from macroscopic matrices S-220.2 vs. S-221.2. The number of days of release are shown on the horizontal axis (X axis). The cumulative percentage of naltrexone release is shown on the vertical axis (Y axis). In particular, **Figure 3A** shows a representative curve for sample release of naltrexone from a matrix without sugar ("S-220.2" Sample ID for the matrix; see Table 1) with a naltrexone payload (on a weight by weight basis; w/w) (shown by lower release curve; release data shown by square-shape points on the lower release curve). **Figure 3A** also shows a representative curve for sample release of naltrexone from a matrix with sugar ("S-221.2" Sample ID for the matrix; see Table 1), with a naltrexone payload (on a weight by weight basis; w/w) (shown by upper release curve; release data shown by diamond-shape points on the upper release curve). Release was measured under conditions of about 37 degrees Celsius. Before release was measured, steps had been taken to incorporate (payload) naltrexone into the matrices. The matrices provide very reliable sustained release of low-dose naltrexone.

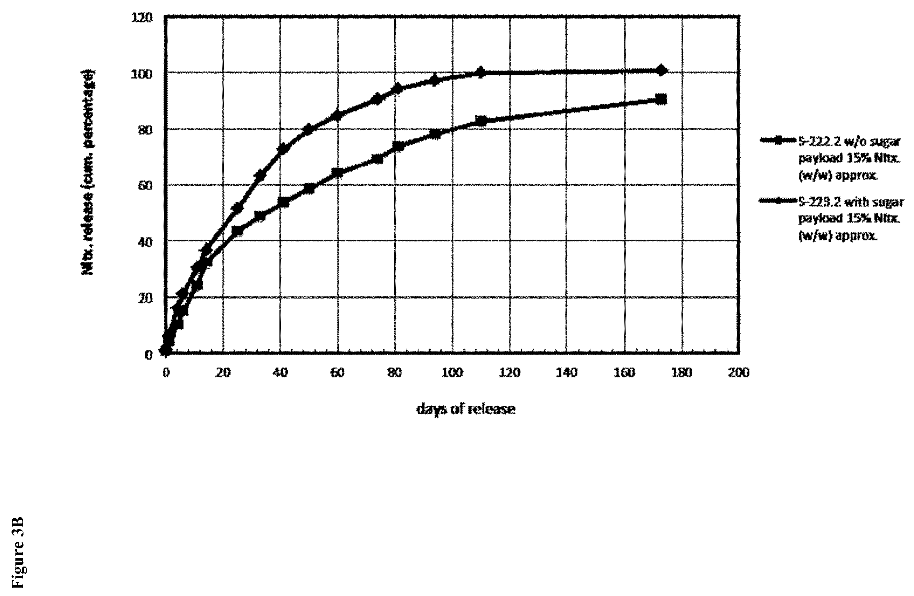

Referring to another non-limiting example, **Figure 3B** shows representative data for naltrexone release from macroscopic matrices S-222.2 vs. S-223.2 (these Sample IDs are shown in Table 1). The number of days of release are shown on the horizontal axis (X axis). The cumulative percentage of naltrexone release is shown on the vertical axis (Y axis). In particular, **Figure 3B** shows a representative curve for sample release of naltrexone from a matrix without sugar ("S-222.2" Sample ID for the matrix; see Table 1), with a naltrexone payload (on a weight by weight basis; w/w) (shown by lower release curve; release data shown by square-shape points on the lower release curve). **Figure 3B** also shows a representative curve for sample release of naltrexone from a matrix with sugar ("S-223.2" Sample ID for the matrix; see Table 1), with a naltrexone payload (on a weight by weight basis; w/w) (shown by upper release curve; release data shown by diamond-shape points on the upper release curve). Before release was measured, steps had been taken to incorporate (payload) naltrexone into the matrices. The matrices provide very reliable sustained release of low-dose naltrexone.

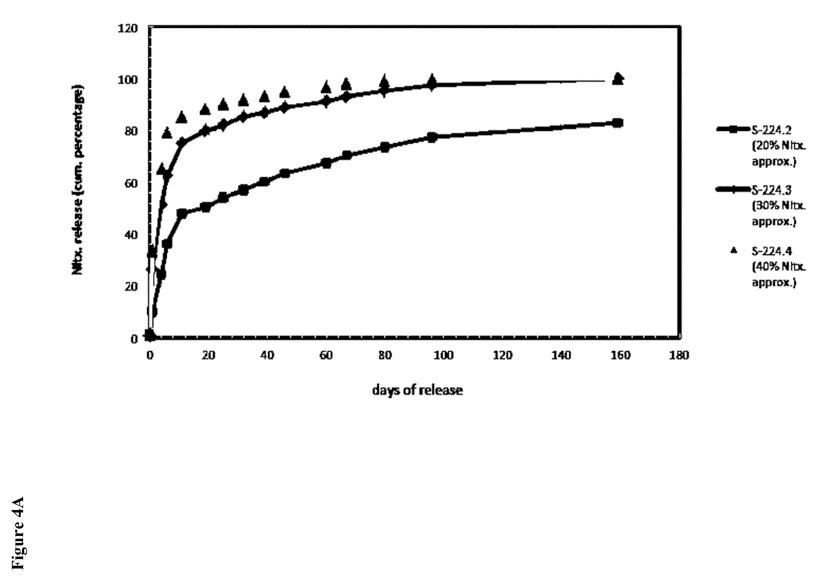

Referring to another non-limiting example, **Figure 4A** shows representative data for naltrexone release from macroscopic matrices S-224.2, S224.3 and 224.4 (these Sample IDs are shown in Table 1). The number of days of release are shown on the horizontal axis (X axis). The cumulative percentage of naltrexone release is shown on the vertical axis (Y axis). In particular, **Figure 4A** shows a representative curve for sample release of naltrexone from the "S-224.2" Sample ID matrix (see Table 1) (shown by lower release curve; release data shown by square-shape points on the lower release curve). **Figure 4A** also shows a representative curve for sample release of naltrexone from the "S-224.3" Sample ID matrix (see Table 1) (shown by middle release curve; release data shown by diamond-shape points on the middle release curve). **Figure 4A** also shows a representative curve for sample release of naltrexone from the "S-224.4" Sample ID matrix (see Table 1) (shown by upper release curve; release data shown by triangle-shape points on the upper release curve). Before release was measured, steps had been taken to incorporate (payload) naltrexone into the matrices. The matrices provide very reliable sustained release of low-dose naltrexone.

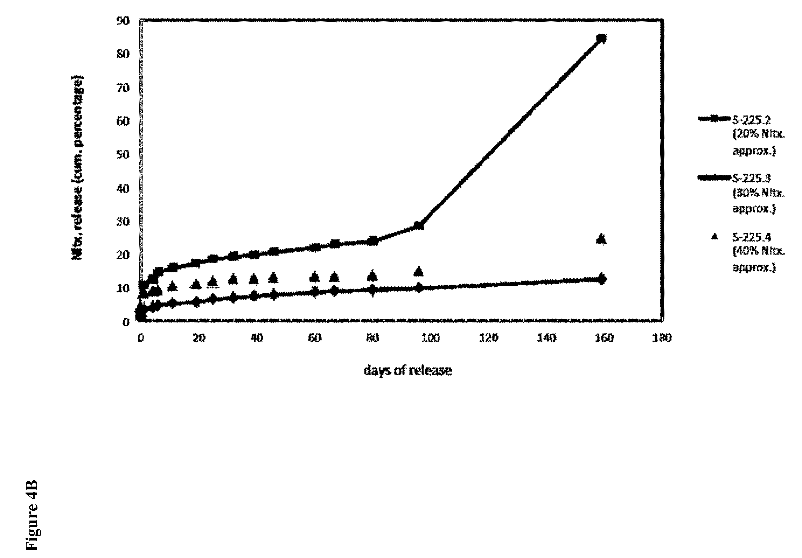

Referring to another non-limiting example, **Figure 4B** shows representative data for naltrexone release from macroscopic matrices S-225.2, S225.3 and 225.4 (these Sample IDs are shown in Table 1). The number of days of release are shown on the horizontal axis (X axis). The cumulative percentage of naltrexone release is shown on the vertical axis (Y axis). In particular, **Figure 4B** shows a representative curve for sample release of naltrexone from the "S-225.2" Sample ID matrix (see Table 1) (shown by upper release curve; release data shown by square-shape points on the upper release curve). **Figure 4B** also shows a representative curve for sample release of naltrexone from the "S-225.3" Sample ID matrix (see Table 1) (shown by lower release curve; release data shown by diamond-shape points on the lower release curve). **Figure 4B** also shows a representative curve for sample release of naltrexone from the "S-225.4" Sample ID matrix (see Table 1) (shown by middle release curve; release data shown by triangle-shape points on the middle release curve). Before release was measured, steps had been taken to incorporate (payload) naltrexone into the matrices. The matrices provide very reliable sustained release of low-dose naltrexone.

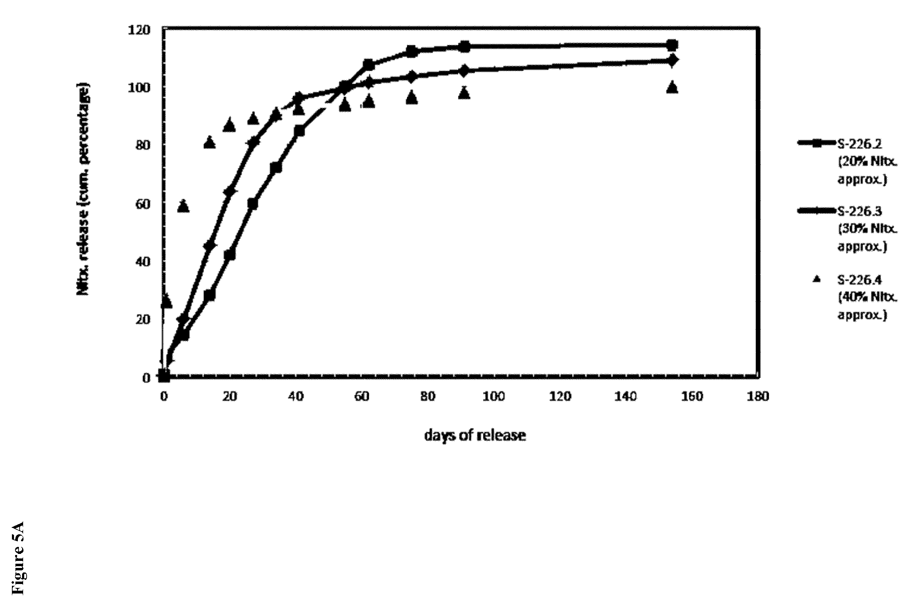

Referring to another non-limiting example, **Figure 5A** shows representative data for naltrexone release from macroscopic matrices S-226.2, S226.3 and 226.4 (these Sample IDs are shown in Table 1). The number of days of release are shown on the horizontal axis (X axis). The cumulative percentage of naltrexone release is shown on the vertical axis (Y axis). **Figure 5A** shows a representative curve for sample release of naltrexone from the "S-226.2" Sample ID matrix (see Table 1) (release data shown by square-shape points on the release curve). **Figure 5A** also shows a representative curve for sample release of naltrexone from the "S-226.3" Sample ID matrix (see Table 1) (release data shown by diamond-shape points on the release curve). **Figure 5A** also shows a representative curve for sample release of naltrexone from the "S-226.4" Sample ID matrix (see Table 1) (release data shown by triangle-shape points on the release curve). Before release was measured, steps had been taken to incorporate (payload) naltrexone into the matrices. The matrices provide very reliable sustained release of low-dose naltrexone.

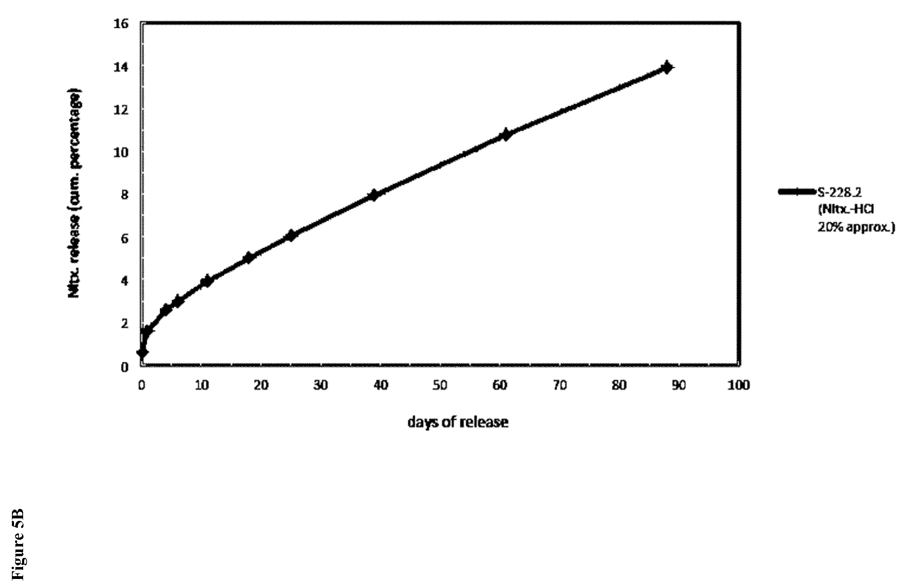

Referring to another non-limiting example, **Figure 5B** shows representative data for naltrexone release from macroscopic matrix S-228.2 (this Sample ID is shown in Table 1) (release data shown by diamond-shape points on the release curve). The number of days of release are shown on the horizontal axis (X axis). The cumulative percentage of naltrexone release is shown on the vertical axis (Y axis). Before release was measured, steps had been taken to incorporate (payload) naltrexone into the matrix. The matrix provides very reliable sustained release of low-dose naltrexone.

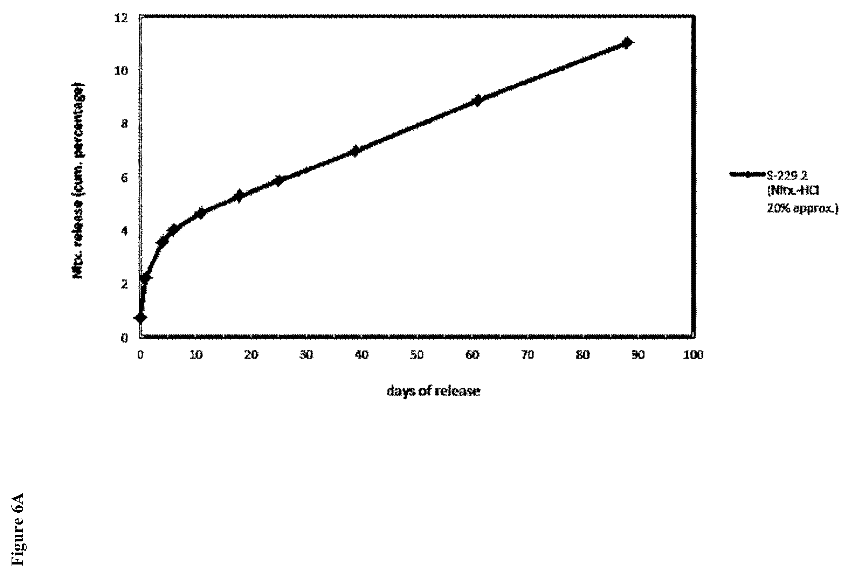

Referring to another non-limiting example, **Figure 6A** shows representative data for naltrexone release from macroscopic matrix S-229.2 (this Sample ID is shown in Table 1) (release data shown by diamond-shape points on the release curve). The number of days of release are shown on the horizontal axis (X axis). The cumulative percentage of naltrexone release is shown on the vertical axis (Y axis). Before release was measured, steps had been taken to incorporate (payload) naltrexone into the matrix. The matrix provides very reliable sustained release of low-dose naltrexone.

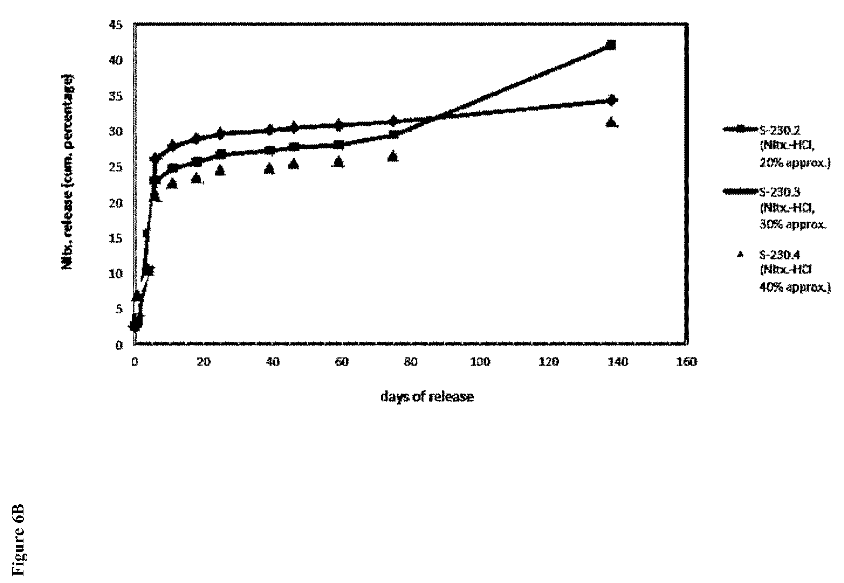

**Figure 6B** shows representative data for naltrexone release from macroscopic matrices S-230.2, S230.3 and 230.4 (these Sample IDs are shown in Table 1). The number of days of release are shown on the horizontal axis (X axis). The cumulative percentage of naltrexone release is shown on the vertical axis (Y axis). **Figure 6B** shows a representative curve for sample release of naltrexone from the "S-230.2" Sample ID matrix (see Table 1) (release data shown by square-shape points on the release curve). **Figure 6B** also shows a representative curve for sample release of naltrexone from the "S-230.3" Sample ID matrix (see Table 1) (release data shown by diamond-shape points on the release curve). **Figure 6B** also shows a representative curve for sample release of naltrexone from the "S-230.4" Sample ID matrix (see Table 1) (release data shown by triangle-shape points on the release curve). Before release was measured, steps had been taken to incorporate (payload) naltrexone into the matrices. The matrices provide very reliable sustained release of low-dose naltrexone.

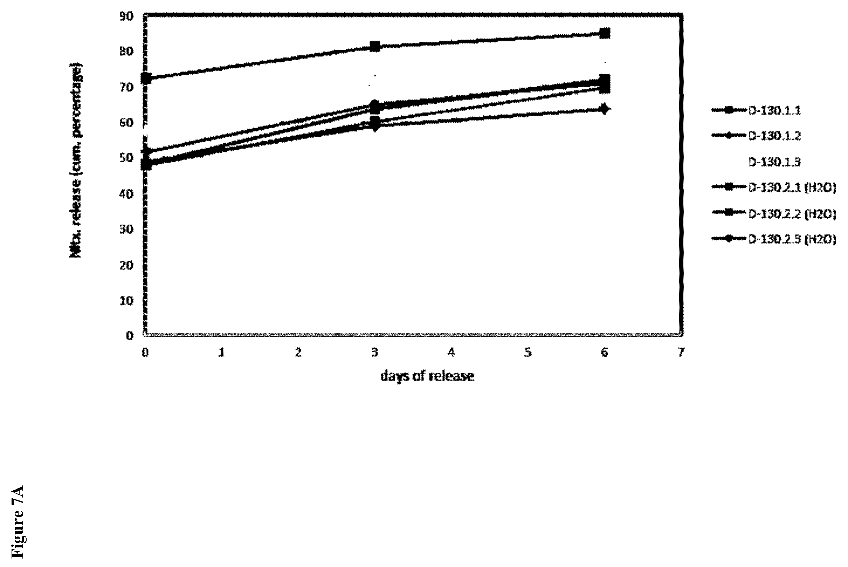

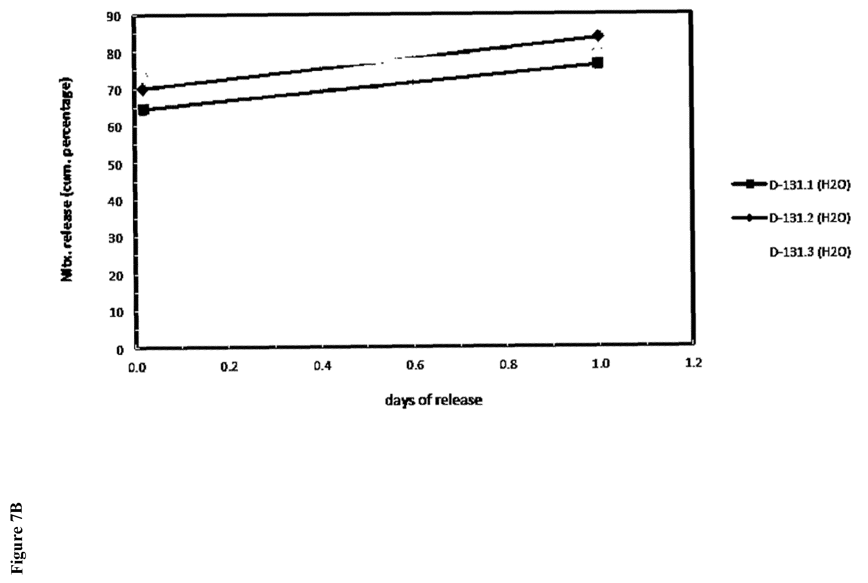

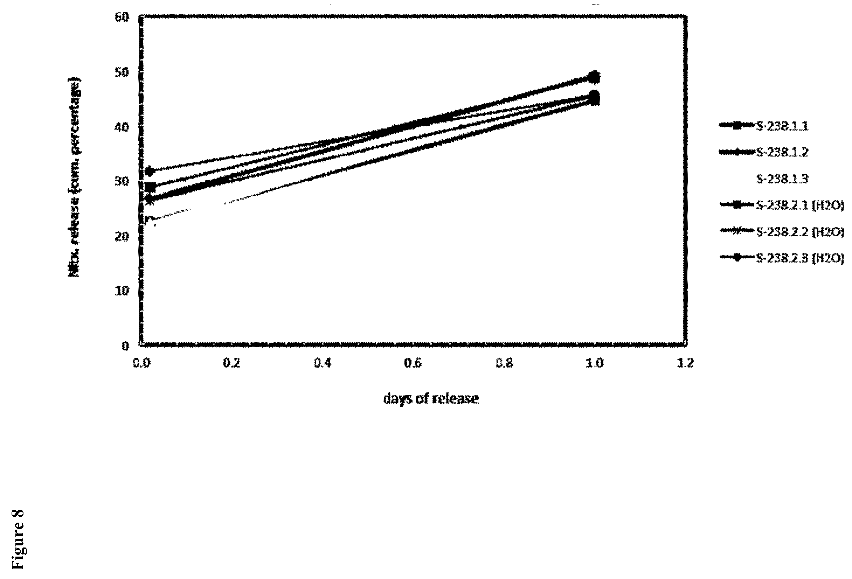

Referring to another non-limiting example, **Table 2** provides a summary of representative micronized matrices, wherein each matrix comprises a naltrexone payload. **Table 2** shows sample ID numbers for each matrix that comprises a naltrexone payload. **Figures 7A****,** **7B** **and** **8** show release data for these sample ID numbers listed in **Table 2.**

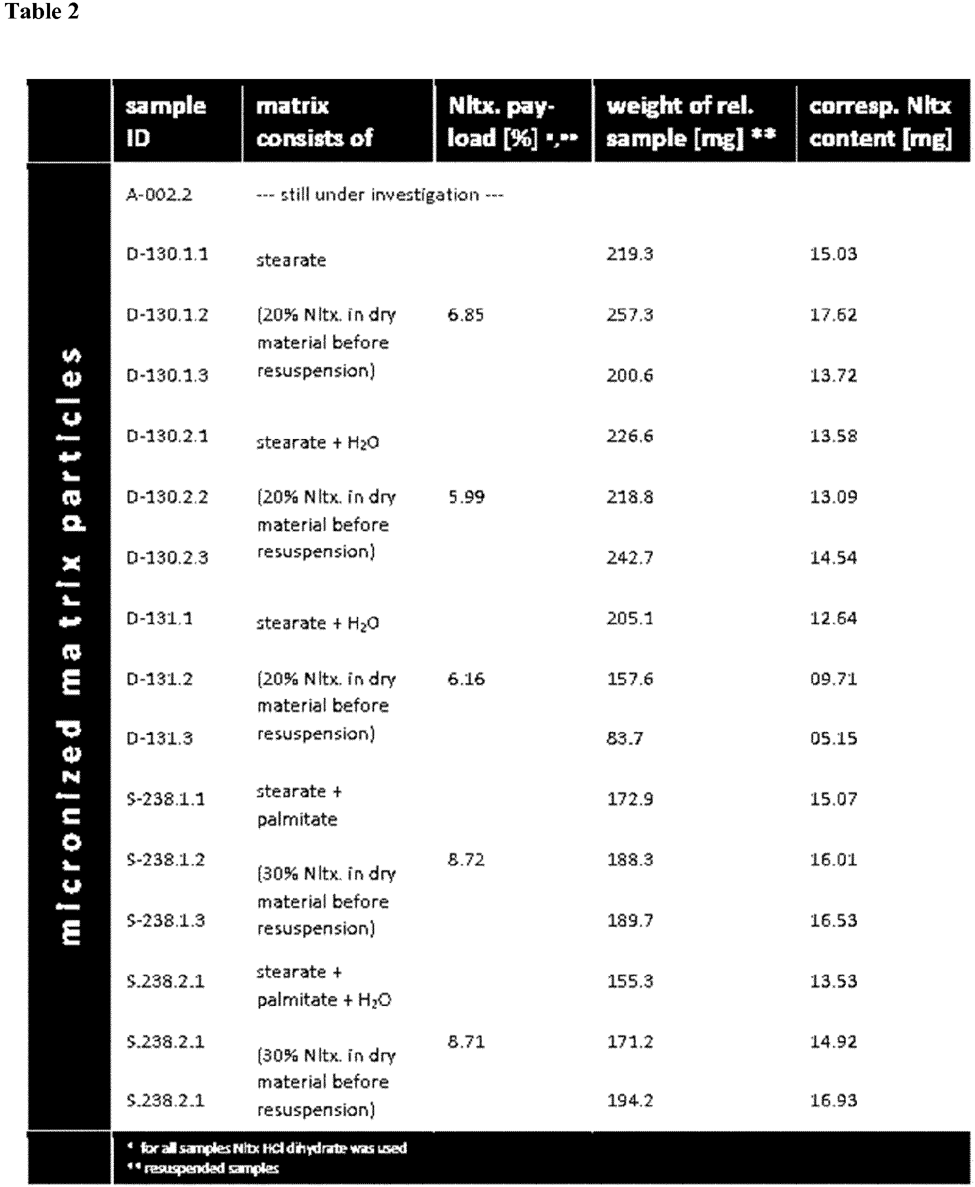

Referring to another non-limiting example, **Figure 7A** shows representative data for naltrexone release from micronized matrices D-130.1.1, D-130.1.2, D-130.1.3, D-130.2.1, D-130.2.2 and D-130.2.3 (these Sample IDs are shown in Table 2). The number of days of release are shown on the horizontal axis (X axis). The cumulative percentage of naltrexone release is shown on the vertical axis (Y axis). By way of example, **Figure 7A** shows a representative curve for sample release of naltrexone from the "D-130.1.1" Sample ID matrix (release data shown by square-shape points on the uppermost release curve). Before release was measured, steps had been taken to incorporate (payload) naltrexone into the matrices. The matrices provide very reliable sustained release of low-dose naltrexone.

Referring to another non-limiting example, **Figure 7B** shows representative data for naltrexone release from micronized matrices D-131.1, D-131.2, and D-131.3 (these Sample IDs are shown in Table 2). The number of days of release are shown on the horizontal axis (X axis). The cumulative percentage of naltrexone release is shown on the vertical axis (Y axis). By way of example, **Figure 7B** shows a representative curve for sample release of naltrexone from the "D-131.1.1" Sample ID matrix (release data shown by square-shape points on the lower release curve). Before release was measured, steps had been taken to incorporate (payload) naltrexone into the matrices. The matrices provide very reliable sustained release of low-dose naltrexone.

Referring to another non-limiting example, **Figure 8** shows representative data for naltrexone release from micronized matrices S-238.1 and S-238.2 (these Sample IDs are shown in Table 2). The number of days of release are shown on the horizontal axis (X axis). The cumulative percentage of naltrexone release is shown on the vertical axis (Y axis). Before release was measured, steps had been taken to incorporate (payload) naltrexone into the matrices. The matrices provide very reliable sustained release of low-dose naltrexone.

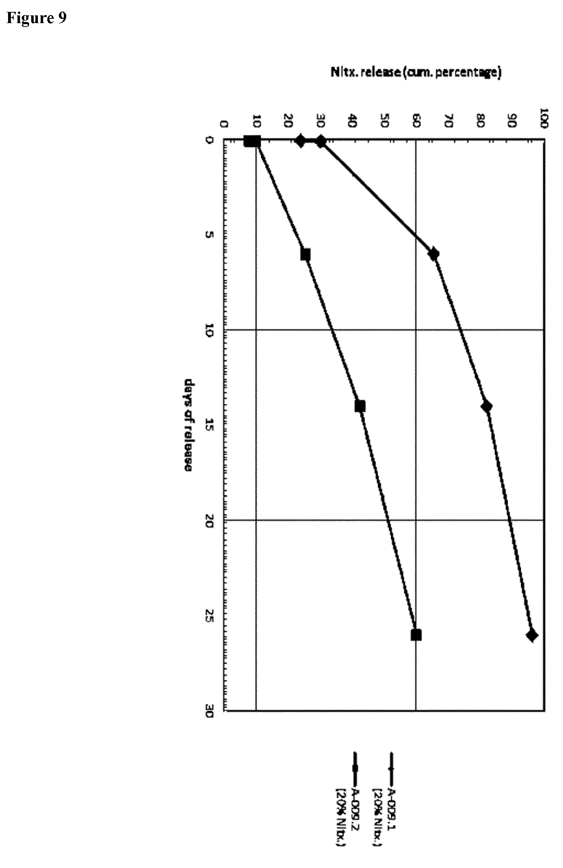

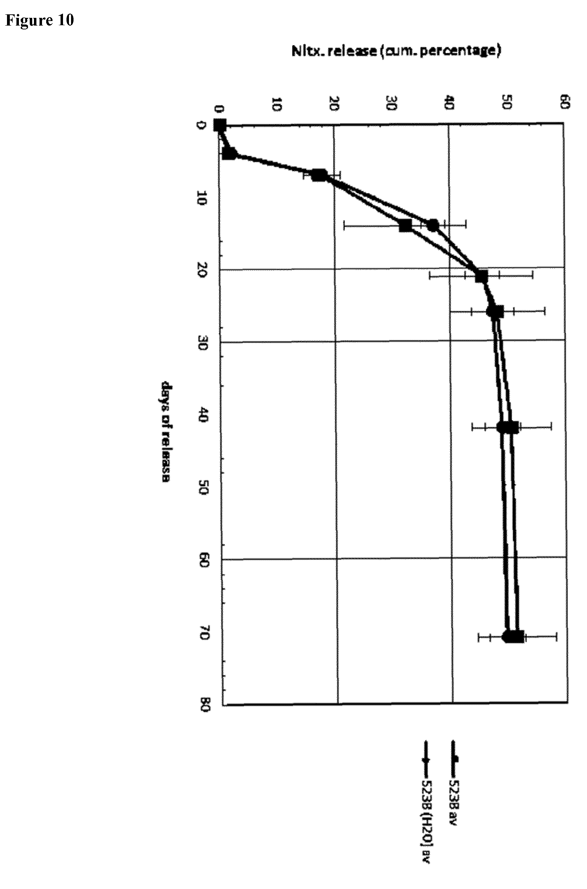

In accordance with the present invention, the incorporation of naltrexone into a matrix can be performed by any suitable method(s) including but not limited to melting, mixing, etc. Additional representative, sample data for naltrexone release is shown in **Figures 9** **and** **10****.**

Referring to another non-limiting example, **Figure 9** shows representative data for naltrexone release from melted stearate matrices (matrix sample IDs A-009.1 and A-009.2) under conditions at a temperature of approximately 37 degrees Celsius. The number of days of release are shown on the horizontal axis (X axis). The cumulative percentage of naltrexone release is shown on the vertical axis (Y axis). Before release was measured, steps had been taken to incorporate (payload) naltrexone into the matrices. The matrices provide very reliable sustained release of low-dose naltrexone.

Referring to another non-limiting example, **Figure 10** shows representative data for naltrexone release from two different matrices (matrix sample IDs S-238.1 and 238.2) in PBS at a temperature of about 37 degrees Celsius (payload 15% naltrexone, approximately). The number of days of release are shown on the horizontal axis (X axis). The cumulative percentage of naltrexone release is shown on the vertical axis (Y axis). Before release was measured, steps had been taken to incorporate (payload) naltrexone into the matrices. The matrices provide very reliable sustained release of low-dose naltrexone.

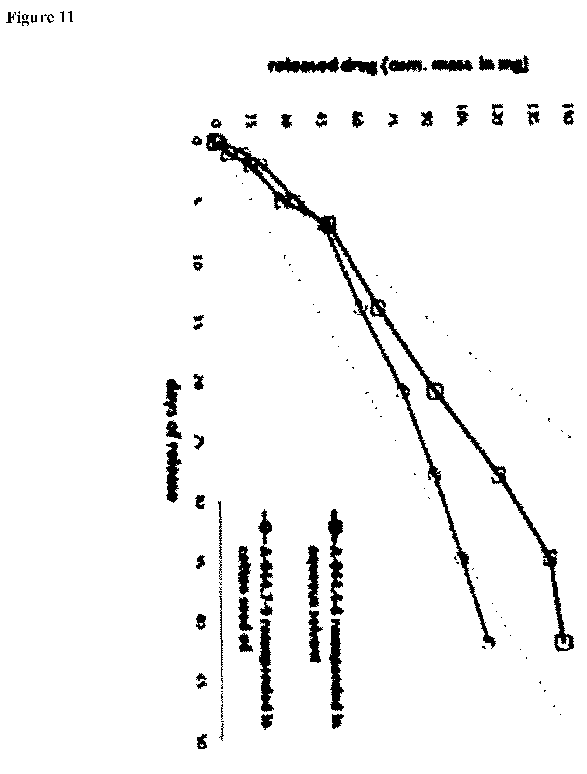

Referring to another non-limiting example, **Figure 11** shows representative data for release of naltrexone base from two different matrices using cottonseed oil vs. water as a solvent to decrease needle gauge. Each matrix included magnesium stearate and tocopherol in a ratio of about 85:15, and with a payload of approximately 40% naltrexone that was incorporated (payloaded) in each matrix. The upper curve (with squares along the curve, representing release at different days) shows release of naltrexone base from a matrix, wherein the matrix had been resuspended in an aqueous solvent. The lower curve (with circles along the curve, representing release at different days) shows release of naltrexone base from a matrix, wherein the matrix had been resuspended in cottonseed oil as a solvent. The number of days of release are shown on the horizontal axis (X axis). The cumulative mass amount of naltrexone release (in mg) is shown on the vertical axis (Y axis). Before release was measured, steps had been taken to incorporate (payload) naltrexone into each matrix. The matrices provide very reliable sustained release of low-dose naltrexone.

The present invention demonstrates that naltrexone base provides release profiles (in contrast to the hydrochloride form) that meet preferred target criteria.

The sustained release formulations of the present invention provide a number of advantages. For instance, there are a number of surprising advantages of using the oil based formulations of the present invention, including the fact that the injection volume can be reduced to below one milliliter with the oil based formulations.

In accordance with the present invention, it has also been surprisingly discovered that the oil based formulations (with oil as solvent) permits not only a reduction of the injection volume, but this also allows one to use a thinner needle because the rheology is improved. For example, instead of using a 22 Gauge needle for a naltrexone formulation in an aqueous suspension, one can advantageously use a 26 or even 27 Gauge needle for the same particles in a formulation using oil as solvent. A mixture step be may applied as needed to achieve the desired results. This represents another significant advantage of the present invention.

While the examples described herein demonstrate sustained release of naltrexone, it is to be understood that the matrices of the present invention are also suitable for achieving sustained release of naloxone in an amount and for a duration that is needed or desired.

It is contemplated that needle size can also be varied in other embodiments to achieve desired results.

The present invention also contemplates other factors including for example, but not limited to, API incorporation from solution (aqueous or alcohol), adaptation of payload to obtain a variation of release duration, reproducibility, improvements in stability, storage testing, and stress release experiments, all of which further demonstrate the advantages of the invention.

In preferred embodiments, the present invention provides sustained release formulations for low dose naltrexone (LDN) such that the LDN can be administered monthly while releasing about 3.5 mg to about 5.5 mg per day. This dosage of naltrexone is considered to be within an appropriate range for treating pain while avoiding nausea. This is also the accepted dose based on clinical trials with the oral preparation.

The sustained release formulations of the present invention also provide surprising and unexpected phannacokinetic advantages, including significantly minimizing fluctuations in plasma levels of naltrexone. Preferably, use of the sustained release formulations of the present invention enables one to essentially avoid fluctuations in plasma levels of naltrexone. The sustained release formulations of the present invention also provide better downregulation of the opioid receptors by avoiding fluctuations in plasma levels.

It is also within the scope of the invention that an injectable sustained release low dose naltrexone formulation of the present invention can be administered with approximately one month to three months of linear delivery, providing about 3 mg to about 5 mg daily release.

The present invention also preferably provides microparticle formulations. An exemplary microparticle formulation, and exemplary methods of preparing such formulations, are described below.

### Example: Manufacture of a naltrexone-containing microparticle formulation

Exemplary Naltrexone Formulation Components:
Magnesium stearate
D,L-alpha tocopherol
Naltrexone
Phosphate buffered saline (PBS)
Hyaluronic acid (to improve injectability)

### Exemplary Protocols for preparing Naltrexone Formulations:

**Exemplary Formula #1** -PBS buffer containing 0.01% sodium azide as suspension and release medium:
   1. To a 1L volumetric flask, add the contents of 1 package of ready-to-use powder for PBS
   2. Add sodium azide (100 µg)
   3. Add Millipore water to the calibration mark
   4. Dissolve all solids
   5. Store at 8 degrees celsius (max)
Exemplary Formula **#2** - Microparticulate powder mass formulated in a one-pot mechanical process
   1. Add inactive and active ingredients according to final formulation composition (magnesium stearate, tocopherol, Naltrexone)
   2. Work the mixture until a microparticulate powder mass is formed
   3. Add a small amount of water or Formula #1 and continue to work the mixture for a few minutes (this aspect will lower the burst effect)
**Exemplary Formula #3** *-* basic matrix formulation
   1. Add inactive (magnesium stearate, tocopherol) ingredients in mass ratio corresponding to macroscopic matrix protocol (2/3 magnesium stearate, 1/3 tocopherol)
   2. Work on ingredient mixture (kneading, pressing, folding) to obtain a kneadable macroscopic mass
**Exemplary Formula #4 - Payloading the matrix**
   1. Combine Formula #3 (all material prepared) with active ingredient (naltrexone)
   2. Work on the mixture (kneading, pressing, folding) to incorporate (payload) naltrexone into the macroscopic matrix. The resulting macroscopic matrix contains a homogeneous distribution of naltrexone
**Exemplary Formula #5 - Micronization of the payloaded matrix**
   1. Add magnesium stearate to Formula #4 to an extent corresponding to final ratio according to standard protocol
   2. Work on the matrix (kneading, pressing, folding) up to microparticulate powder has formed supported by anti-cake property of magnesium stearate
**Exemplary Formula #6 -** Fractionation if needed (for example, sieving)

### Other Exemplary Formulation Procedure

This includes a formulation procedure for injectable microparticle suspensions based on a matrix composition (magnesium stearate, tocopherol or plant oil plus payload).
1. The final formulation amounts of all active and passive ingredients are weighed and put into one and the same reaction vessel (one-pot reaction).
   The composition contains an excess of magnesium stearate.
   The stearate is used twofold, to constitute the delivery matrix itself (glued together by tocopherol or plant oil) and to stabilize mechanically formed new surface areas by anticake properties. The anti-cake effect remembers strongly the so-called Rehbinder-effect being a powerful surfactant-supported process to stabilize newly formed solid surfaces or to make the formation of new surface areas easier.
2. Work on the final composition mechanically (kneading, pressing, folding, grinding, milling). This leads after sustained mechanical processing to a more or less steady state of microparticle distribution. Processes of simultaneous formation and destroying (by aggregation/fusion/coalescence) of microparticles balance each other and provide a stationary microparticle size distribution. The microparticle size distribution reflects two types of particle distributions: pure magnesium stearate (smaller diameter fraction) and payloaded magnesium stearate/hydrophobic glue/drug (naltrexone) particles (larger diameter particles). The formed payloaded particles have a size and size distribution to guarantee the injection via a given inner needle diameter (Gauge number). If needed a fractionation can be utilized. As part of the formulation, the particle powder is resuspended, either in an aqueous or an oily environment, and this involves mechanical impetus.
3. Aliquots of the powder can be taken for pre-filling the syringes or for forming microparticle suspensions outside of the syringes.
4. Pre-filled syringes can contain a biocompatible oil or saline or saline with hyaluronic acid to make injection through a needle easier.

### Additional Example of a Sustained Release Low Dose Naltrexone (LDN) Formulation:

- Subcutaneous formulation for injection as depot or suspension, or reconstituted in preloaded syringes
- Concentration of LDN at 150mg/mL formulation
- Release duration as follows:
   ∘ One month with 4.5mg (range 2.5mg to 6.5mg) release per day
   ∘ At least 80% released over delivery duration
   ∘ Achieves an injection volume of not more than 3 mL as suspension or depot
   ∘ Bioavailability greater than 70% through release period
   ∘ Fully absorbed without being palpable under the skin

The formulations of the present invention can be adjusted as needed or desired to provide desired shelf life, stability, and safety profiles and characteristics (including at pre-clinical and launch stages).

Manufacture of the formulations of the present invention also provides additional advantages. Manufacture can be performed at very low incremental costs; and manufacture can be generally automated. Throughout manufacture, according to preferred embodiments, the biological activity of the API (e.g. naloxone or naltrexone) is not affected. All the manufacturing processes and test methods can be fully validated under QSR (GMP) and CE to ensure purity, dose, and all other properties are fully controlled.

Alternative materials are possible in the formulation process.

The present invention provides a number of additional surprising and unexpected advantages, which are achieved by using the formulations described herein. These advantages include, but are not limited to, addressing and reducing the severity of numerous problems that currently exist with conventional approaches. These problems that currently exist with conventional approaches include, but are not limited to, the following:
(1) Fibromyalgia and chronic pain patients often have very poor compliance with oral medications, and therefore a physician-administered injection, in accordance with the present invention, can significantly improve patient outcomes; and
(2) Many patients on conventional oral LDN (oral low dose naltrexone) report regular nausea and other complications. The LDN formulations of the present invention help reduce these complications.

Therapy decisions about other treatments for a given patient will also be simplified since the LDN (based on administration by the methods of the present invention) will be steady state, or substantially steady-state, in the patient. Also, using an injectable LDN of the present invention, the treating physician will also be in control of the therapy.

Moreover, according to the present invention, the effects of LDN can be improved by going from a daily tablet to a monthly injection. Also, patients who respond well to a one-month formulation can be moved to a two or three-month version, simplifying therapy.

While the present invention can be used for treatment and reducing the severity of symptoms in fibromyalgia, the formulations of the present invention can also be used in the treatment or reducing the severity of other chronic conditions including but not limited to Crohn's disease, multiple sclerosis and pruritus associated systemic sclerosis.

Furthermore, the present invention contemplates that naloxone or naltrexone formulations can be administered by means including but not limited to subcutaneous, intraperitoneal, intramuscular, or transdermal administration, or by other routes.

The present invention also contemplates useful and beneficial sustained release formulations of acamprosate. The present invention also contemplates that acamprosate formulations can be administered by means including but not limited to subcutaneous, intraperitoneal, intramuscular, or transdermal administration.

The foregoing descriptions of certain embodiments of the present invention have been presented for purposes of illustration and description. The embodiments described herein are not intended to be exhaustive or to limit the scope of the present invention in any way.

The invention is also characterized by the following items.
1. A drug delivery composition, comprising:
   a microparticulate formulation, said microparticulate formulation further comprising:
      a matrix, a solvent, and at least one low dose opioid receptor antagonist or a combination thereof, wherein said opioid receptor antagonist is incorporated into said matrix homogenously or substantially homogenously.
2. The drug delivery composition of item 1, wherein the matrix controls release of said opioid receptor antagonist for initial release rate, daily release rate, duration, and completeness of release, allows release of exact amount of the opioid receptor antagonist, allows sustained release of the opioid receptor antagonist, or a combination thereof.
3. The drug delivery composition of item 1 or 2, wherein the matrix comprises a solid hydrophobic component and a liquid hydrophobic component.
4. The drug delivery composition of any of items 1 to 3, wherein the solvent is phosphate buffered saline or water.
5. The drug delivery composition of any of items 1 to 4, wherein the low dose opioid receptor antagonist is selected from group consisting of naltrexone and naloxone.
6. The drug delivery composition of any of items 1 to 5, wherein the microparticulate formulation is in a powder form or an injectable form.
7. A method of manufacturing the drug delivery composition of item 3, comprising:
   mixing the solid hydrophobic component and the liquid hydrophobic component of the matrix and the opioid receptor antagonist until a microparticulate formulation in a powder form is formed;
   adding a small amount of water or phosphate buffered saline; and
   mixing continuously to incorporate the opioid receptor antagonist homogenously or substantially homogenously into the matrix.
8. A method of manufacturing the drug delivery composition of item 3, comprising:
   adding the solid hydrophobic component and the liquid hydrophobic component of the matrix;
   mixing said components by kneading, pressing, folding or combination thereof to obtain a kneadable macroscopic mass;
   combining the macroscopic mass with the opioid receptor antagonist;
   mixing the macroscopic mass with the opioid receptor antagonist by kneading, pressing, folding or a combination thereof to incorporate the opioid receptor antagonist homogenously into the matrix, thereby payloading the matrix;
   micronizing the payloaded matrix by adding magnesium stearate to the payloaded matrix and mixing the payloaded matrix and magnesium stearate by kneading, pressing, folding or a combination thereof until a microparticulate formulation in a powder form is formed.
9. The method of item 8, wherein said method further comprises:
   fractionating the microparticulate formulation by sieving.
10. A method to manufacture the drug delivery composition of item 3, comprising:
   adding the solid hydrophobic component and liquid hydrophobic component of the matrix and the opioid receptor antagonist in a reaction vessel, wherein the solid hydrophobic component is added in a two-fold amount to the mixture; and
   mixing the components and the opioid receptor antagonist by kneading, pressing, folding, grinding, milling, or a combination thereof so that the microparticulate formulation has a size and size distribution that allows it to be administered as an injectable.
11. The method of item 10, further comprising:
   fractioning the microparticulate formulation by sieving.
12. The method of item 10, wherein said microparticulate formulation is resuspended in an aqueous or an oily suspension.
13. The method of item 10, wherein the microparticulate formulation is prefilled in a syringe in powder form or dissolved in aqueous solution outside of the syringe and then used to fill the syringe.
14. The method of item 13, wherein the prefilled syringe further comprises at least one of a biocompatible oil, saline, and saline with hyaluronic acid.
15. A method of treating an individual with a chronic pain disorder, comprising:
   administering the drug delivery composition of any of claims 1 to 7 to the individual.
16. The method of item 15, wherein the drug delivery composition is administered intramuscularly, intravenously, intradermally, subcutaneously, or via another route.
17. The method of item 15, wherein said administration allows sustained release of the opioid receptor antagonist in the individual's body.
18. The method of item 17, wherein the individual is suffering from or has been diagnosed with fibromyalgia, Crohn's disease, multiple sclerosis, and pruritus associated with systemic sclerosis.

## Claims

1. A drug delivery composition, comprising:
a microparticulate formulation, said microparticulate formulation further comprising:
a matrix, a solvent, and at least one low dose opioid receptor antagonist or a combination thereof, wherein said opioid receptor antagonist is incorporated into said matrix homogenously or substantially homogenously.

2. The drug delivery composition of claim 1, wherein the matrix controls release of said opioid receptor antagonist for initial release rate, daily release rate, duration, and completeness of release, allows release of exact amount of the opioid receptor antagonist, allows sustained release of the opioid receptor antagonist, or a combination thereof.

3. The drug delivery composition of claim 1, wherein the matrix comprises a solid hydrophobic component and a liquid hydrophobic component.

4. The drug delivery composition of claim 1, wherein the solvent is phosphate buffered saline or water.

5. The drug delivery composition of claim 1, wherein the low dose opioid receptor antagonist is selected from group consisting of naltrexone and naloxone.

6. The drug delivery composition of claim 1, wherein the microparticulate formulation is in a powder form or an injectable form.

7. A method of manufacturing the drug delivery composition of claim 3, comprising:
mixing the solid hydrophobic component and the liquid hydrophobic component of the matrix and the opioid receptor antagonist until a microparticulate formulation in a powder form is formed;
adding a small amount of water or phosphate buffered saline; and
mixing continuously to incorporate the opioid receptor antagonist homogenously or substantially homogenously into the matrix.

8. A method of manufacturing the drug delivery composition of claim 3, comprising:
adding the solid hydrophobic component and the liquid hydrophobic component of the matrix;
mixing said components by kneading, pressing, folding or combination thereof to obtain a kneadable macroscopic mass;
combining the macroscopic mass with the opioid receptor antagonist;
mixing the macroscopic mass with the opioid receptor antagonist by kneading, pressing, folding or a combination thereof to incorporate the opioid receptor antagonist homogenously into the matrix, thereby payloading the matrix;
micronizing the payloaded matrix by adding magnesium stearate to the payloaded matrix and mixing the payloaded matrix and magnesium stearate by kneading, pressing, folding or a combination thereof until a microparticulate formulation in a powder form is formed.

9. The method of claim 8, wherein said method further comprises:
fractionating the microparticulate formulation by sieving.

10. A method to manufacture the drug delivery composition of claim 3, comprising:
adding the solid hydrophobic component and liquid hydrophobic component of the matrix and the opioid receptor antagonist in a reaction vessel, wherein the solid hydrophobic component is added in a two-fold amount to the mixture; and
mixing the components and the opioid receptor antagonist by kneading, pressing, folding, grinding, milling, or a combination thereof so that the microparticulate formulation has a size and size distribution that allows it to be administered as an injectable.

11. The method of claim 10, further comprising:
fractioning the microparticulate formulation by sieving.

12. The method of claim 10, wherein said microparticulate formulation is resuspended in an aqueous or an oily suspension.

13. The method of claim 10, wherein the microparticulate formulation is prefilled in a syringe in powder form or dissolved in aqueous solution outside of the syringe and then used to fill the syringe.

14. The method of claim 13, wherein the prefilled syringe further comprises at least one of a biocompatible oil, saline, and saline with hyaluronic acid.

15. The use of the drug delivery composition of claim 1 for treating an individual with a chronic pain disorder, comprising:
administering the drug delivery composition of claim 1 to the individual,
wherein the drug delivery composition is preferably administered intramuscularly, intravenously, intradermally, subcutaneously, or via another route, or
wherein said administration preferably allows sustained release of the opioid receptor antagonist in the individual's body, or
wherein the individual is preferably suffering from or has been diagnosed with fibromyalgia, Crohn's disease, multiple sclerosis, and pruritus associated with systemic sclerosis.
